# EUROPEAN PATENT SPECIFICATION

(11) **EP 1 512 427 B1**
(45) Date of publication and mention of the grant of the patent: **02.01.2008**
(21) Application number: 04254972.5
(22) Date of filing: 18.08.2004
(51) Int. Cl.: A61M 27/00, A61M 25/00

(54) **Trimmable sensing catheter**
Abgleichbarer Messkatheter
Cathéter ajustable de détection

(30) Priority: 18.08.2003 US 642772
(43) Date of publication of application: 09.03.2005
(73) Proprietor: Codman & Shurtleff, Inc., Raynham, Massachusetts 02767-0350 (US)
(72) Inventor: Rosenberg, Meir, Newton, MA 02459 (US)
(74) Representative: Mercer, Christopher Paul

(56) References cited:
- EP-A- 1 040 844
- US-A- 5 191 898
- US-A- 5 957 912
- US-A1- 2003 130 620
- US-B1- 6 210 346
- US-B1- 6 295 877

## Description

### FIELD OF THE INVENTION

The present invention relates to an implantable fluid management device useful with a shunt system, and in particular it features a trimmable hydrocephalus catheter having a pressure sensor disposed therein.

### BACKGROUND OF THE INVENTION

Hydrocephalus is a neurological condition that is caused by the abnormal accumulation of cerebrospinal fluid (CSF) within the ventricles, or cavities, of the brain. CSF is a clear, colorless fluid that is primarily produced by the choroid plexus and surrounds the brain and spinal cord. CSF constantly circulates through the ventricular system of the brain and is ultimately absorbed into the bloodstream. CSF aids in the protection of the brain and spinal cord. Because CSF keeps the brain and spinal cord buoyant, it acts as a protective cushion or "shock absorber" to prevent injuries to the central nervous system.

Hydrocephalus, which affects children and adults, arises when the normal drainage of CSF in the brain is blocked in some way. Such blockage can be caused by a number of factors, including, for example, genetic predisposition, intraventricular or intracranial hemorrhage, infections such as meningitis, head trauma, or the like. Blockage of the flow of CSF consequently creates an imbalance between the amount of CSF produced by the choroid plexus and the rate at which CSF is absorbed into the bloodstream, thereby increasing pressure on the brain, which causes the ventricles to enlarge.

Hydrocephalus is most often treated by surgically inserting a shunt system that diverts the flow of CSF from the ventricle to another area of the body where the CSF can be absorbed as part of the circulatory system. Shunt systems come in a variety of models, and typically share similar functional components. These components include a ventricular catheter which is introduced through a burr hole in the skull and implanted in the patient's ventricle, a drainage catheter that carries the CSF to its ultimate drainage site, and optionally a flow-control mechanism, e.g., shunt valve, that regulates the one-way flow of CSF from the ventricle to the drainage site to maintain normal pressure within the ventricles. The ventricular catheter typically contains multiple holes or pores positioned along the length of the ventricular catheter to allow the CSF to enter into the shunt system. To facilitate catheter insertion, a removable rigid stylet, situated within the lumen of the ventricular catheter, is used to direct the catheter toward the desired targeted location. Alternatively, or in addition, blunt tip brain cannulas and peel-away sheaths have been used to aid placement of the catheters.

One common problem encountered with the use of ventricular catheters is the difficulty in measuring the pressure within the patient's ventricle. Many pressure sensors are available for measuring pressure, and these systems typically include a pressure-sensing element in communication with an electronic component. The electronic component is energized by an extra-corporeal energy source which transfers energy through an antenna which is part of the implant. The antenna usually serves to transmit data from the implant to the external interrogating device. Ventricular catheters can contain pressure sensors, however, the pressure-sensing element must be very small due to the size constraints within the ventricle. As a result, the ability to energize the sensor is limited. Accordingly, the use of any sensor with a ventricular catheter will require a tethered system, wherein a wire runs from the sensor to an antenna that is positioned at a location remote from the catheter. The use of a wire, however, will require the catheter to have a fixed length since cutting of the catheter would break the connection in the wires. These catheters, as a result, can only be made in a unitized fashion, requiring stocking of assemblies in various lengths. The extra length of the catheter can also make insertion more difficult. The features of this prior art system form the basis for the preamble of claim 1 appended hereto.

Accordingly, there remains a need for a catheter which can be trimmed to a desired length, and which includes a sensor disposed therein.

### SUMMARY OF THE INVENTION

The present invention provides an implantable fluid management device as defined in claim 1 having an elongate catheter with a proximal end, a distal end, and a first inner lumen extending therethrough for correction to as implantable valve device, and a sensor disposed at a distal portion of the catheter. The device also includes at least one wire having a distal end coupled to the sensor and having a proximal end that is adapted to mate to an external component for powering and/or communicating with the sensor. The at least one wire extends along a length of the catheter such that the at least one wire is in fluid isolation from the inner lumen of the catheter, and characterised in that it is separable from a proximal portion of the catheter whereby the length of the catheter is selectively adjustable.

In one embodiment, the at least one wire can be disposed within a second lumen that is isolated from the first lumen. The second lumen can be formed within an invagination of the outer wall of the catheter extending within the first lumen. In an exemplary embodiment, the first lumen has a diameter that is greater than a diameter of the second lumen. The device can also include a slit extending through an outer wall of the catheter into the second lumen. The slit preferably extends along at least a portion of a length of the catheter from the proximal end thereof such that a portion of the at least one wire can be at least partially removed from the catheter through the slit to allow the length of the catheter to be selectively adjusted. In an exemplary embodiment, the slit extends along a distance less than the length of the catheter, and more preferably the slit extends along less than about one half of the length of the catheter. The slit can be substantially fluid impermeable in a closed position and/or the catheter can be made from a material that is self-sealing.

In another embodiment, the at least one wire is disposed within a second lumen that is isolated from the first lumen and the slit extends into the second lumen. Alternatively, the at least one wire can be disposed within a secondary catheter that is coupled to the catheter. The secondary catheter is preferably adapted to be peeled apart from the catheter to allow the length of the catheter to be selectively adjustable, independent of the length of the secondary catheter.

### BRIEF DESCRIPTION OF THE DRAWINGS

The invention will be more fully understood from the following detailed description taken in conjunction with the accompanying drawings, in which:

FIG. is a perspective view of one embodiment of a ventricular catheter according to the present invention;

FIG. 2 is a cross-sectional view taken across line 2-2 of the ventricular catheter shown in FIG. 1;

FIG. 3 is a perspective view of the ventricular catheter shown in FIG. 1 having a portion of the wire removed therefrom;

FIG. 4 is a perspective view of the catheter and wire shown in FIG. 3 having a portion of the catheter removed therefrom; and

FIG. 5 is a cross-sectional view of another embodiment of a ventricular catheter according to the present invention.

### DETAILED DESCRIPTION OF THE INVENTION

The present invention generally provides an implantable fluid management device that includes a catheter having at least one wire running therethrough, which is coupled to a sensor disposed at a distal portion of the catheter. At least a portion of the wire is removably coupled to the catheter to allow the length of the catheter to be selectively adjusted, thereby providing a trimmable sensing catheter. The device can be used for a variety of medical procedures, but in an exemplary embodiment the device is a ventricular catheter that is used to drain CSF from a patient's ventricles.

The fluid management device is particularly advantageous in that it provides a catheter having a sensor and wires running therethrough, yet it can be trimmed to a desired length without affecting the operability of the wire(s). Current sensing catheters cannot be cut to a desired length, as this would result in a breakage of the wire connection. With ventricular catheters, the necessary length of the catheter cannot be determined until the catheter is implanted, thus making it desirable to provide a catheter having an adjustable length. Accordingly, the present invention advantageously provides a trimmable sensing catheter.

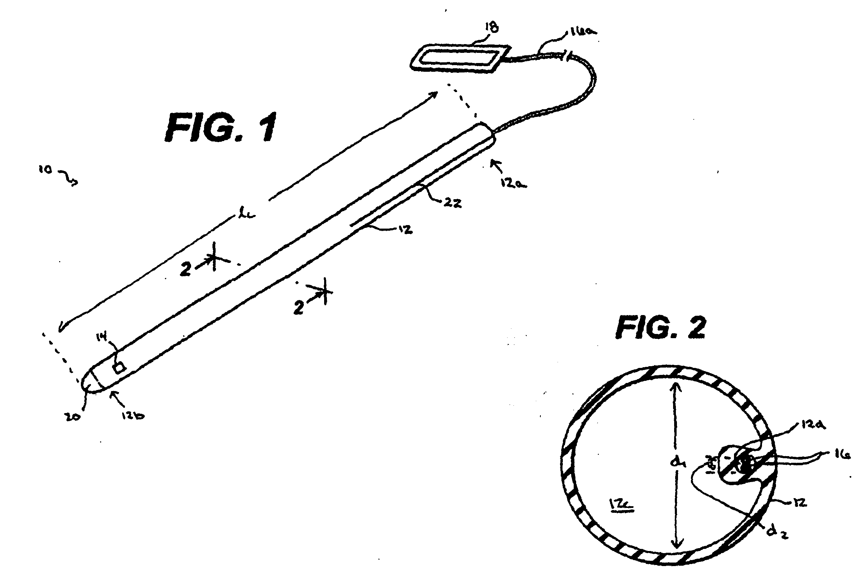

FIG. 1 illustrates an exemplary embodiment of an implantable fluid management device 10 having an elongate catheter 12 with a proximal end 12a, a distal end 12b, and at least one inner lumen 12c (FIG. 2) extending therethrough. A sensor 14 can be disposed at a distal portion of the catheter 12. As shown in FIG. 2, the device 10 also includes at least one wire 16 having a distal end (not shown) coupled to the sensor 14 and having a proximal end 16a that is adapted to mate to an external component, such as an antenna 18, for powering and/or communicating with the sensor 14. The at least one wire 16 extends along a length of the catheter 12 such that the at least one wire 16 is in fluid isolation from the inner lumen 12c of the catheter 12, and it is separable from a proximal portion 12a of the catheter 12 such that the length of the catheter 12 is selectively adjustable.

The elongate catheter 12 can have a variety of configurations, but it is preferably a semi-flexible or flexible elongate member having proximal and distal ends 12a, 12b with at least one inner lumen 12c extending therebetween. The proximal end 12a is preferably open and it can be adapted to connect to another medical device, such as a valve for controlling fluid flow from the catheter. The distal end 12b, on the other hand, can either be open or closed, but preferably it is closed and includes a blunt end cap 20 formed thereon to facilitate insertion and/or imaging of the device 10. The end cap 20 is advantageous in that it facilitates insertion of the device and it prevents the distal tip of an insertion device, such as a rigid stylet (not shown), from penetrating the distal end 12b of the catheter 12. The end cap 20 can also optionally be formed from a radio-opaque material to facilitate imaging of the catheter 12. The catheter 12 can also include one or more fluid-entry ports (not shown) formed in the sidewall thereof and in communication with lumen 12c to allow fluid to flow into the catheter 12.

The dimensions of the catheter 12 can also vary depending on the intended use, but preferably the catheter 12 has a length *l_{c}* that is sufficient to allow at least the distal portion 12b of the catheter 12 to be implanted in a patient's ventricles, while the proximal portion 12a can extend therefrom. The catheter 12 should, however, include excess length to allow the catheter 12 to be trimmed to the appropriate size after implantation of the distal portion 12b of the catheter 12 in the patient's ventricles. In an exemplary embodiment, the length *l_{c}* is in the range of about 10 cm to 20 cm, and more preferably it is about 15 cm.

A person skilled in the art will appreciate that the catheter 12 can have virtually any configuration, shape, and size, and that it can be adapted for use in a variety of medical procedures.

The device 10 also includes a sensor 14 disposed at a distal end of the catheter 12 for measuring and/or communicating conditions present within and/or around the catheter 12. The sensor 14 can be disposed on any portion of the catheter 12, and virtually any type of sensor can be used with the device 10. In an exemplary embodiment, however, the sensor 14 is preferably a pressure sensor that is adapted to measure the pressure present around and/or within the catheter 12, and more preferably the sensor 14 is positioned at a location where it is effective to measure the pressure within the patient's ventricles, rather then the pressure within the lumen 12c of the catheter 12. This is desirable as the fluid flow through the catheter lumen 12c is not always indicative of the pressure within the ventricles. For example, blockage can occur in the fluid-entry ports in the catheter 12 as a result of tissue ingrowth or debris, thereby hindering the flow of fluid into the catheter 12. Accordingly, the pressure sensor 14 is preferably disposed on an external surface of the catheter 12, or it is embedded within the walls and/or end cap 20 of the catheter 12 such that it is effective to measure the pressure surrounding the catheter 12. While virtually any sensor can be used, suitable sensors can be obtained from Millar, of Houston, Texas. A person skilled in the art will appreciate that virtually any sensor can be used to sense a variety of conditions.

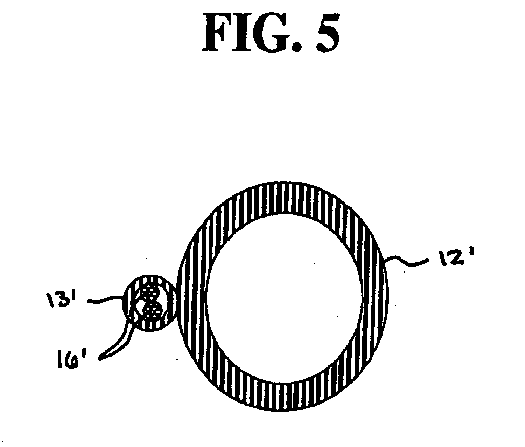

The device 10 further includes at least one wire 16 having a distal end (not shown) that is mated to the sensor 14, and a proximal end 16a that extends from the proximal end 12a of the catheter 12 and that is adapted to couple to an external component for powering and/or communicating with the sensor, such as antenna 18 which receives energy to power the sensor 14. The wire(s) 16 can be disposed in any portion of the catheter 12, but it should be in fluid isolation from the inner lumen 12c of the catheter 12 to prevent the wire(s) 16 from corroding or otherwise interfering with use of the device 10. In one embodiment (not shown), the wire(s) 16 can include a protective coating disposed thereon for protecting the wire(s) 16 from any fluid flowing through the lumen 12c. In another embodiment, shown in FIG. 5, the wire(s) 16' can be disposed within a separate catheter 13' that is mated to catheter 12' in a way that will allow the second catheter 13' to be peeled apart from the first catheter 12', thus allowing the length of the catheter 12' to be selectively adjusted.

In an exemplary embodiment, however, the wire(s) 16 are embedded in the wall of the catheter 12 such that they are disposed within a second lumen 12d that is separate from the first lumen 12c, as shown in FIG. 2. The second lumen 12d, which should extend from the sensor 14 through the entire length of the catheter 12, can be formed using a variety of techniques. In one embodiment, the second lumen 12d can be formed by extruding the catheter 12 around the wire(s) 16 during manufacturing, e.g., as an invagination of the outer wall of the catheter 12 extending within the first lumen 12c. Alternatively, the second lumen 12d can be formed as an actual lumen 12d that is adapted to later receive wire(s) 16 therein. Regardless of the manufacturing technique, the second lumen 12d preferably has a diameter *d₂* that is substantially less than a diameter *d₁* of the first lumen 12c to allow a sufficient amount of fluid to flow through the first lumen 12c without interference from the second lumen 12d, which may protrude somewhat into the first lumen 12c, as shown. In an exemplary embodiment, the diameter *d₁* of the first lumen 12c is in the range of about 1.0 mm to 2.0 mm, and more preferably it is about 1.5 mm, and the diameter *d₂* of the second lumen is in the range of about 50 µm to 250 µm. A person skilled in the art will appreciate that a variety of other techniques can be used to couple the wire(s) 16 to the catheter 12 such that they are at least temporarily separable from the catheter 12 to allow the catheter 12 to be trimmed.

The proximal end 16a of the wire(s) 16 can mate to a variety of external components for powering and/or communicating with the sensor 14. In an exemplary embodiment, however, the wire(s) 16 are mated to an external antenna 18 for receiving power to energize the sensor 14. The antenna 18 can have virtually any configuration, but it is preferably adapted to be implanted at a location within the patient's body that is adjacent to the implant site of the catheter 12. Where the catheter 12 is used as a ventricular catheter, the antenna 18 can, for example, be implanted between the patient's scalp and skull. The use of an external antenna 18 for receiving energy advantageously allows the use of a sensor 14 having a relatively small size.

As previously stated, the device 10 also includes a technique that allows at least a portion of the wire(s) to be separated from the catheter 12 to allow the catheter 12 to be cut. While a variety of techniques can be used to provide this feature, in one embodiment the catheter 12 can include a slit 22 formed therein for allowing the wire(s) 16 to be passed through the slit 22. In an exemplary embodiment, the slit 22 extends through the wall of the catheter 12 such that it is in communication with the second inner lumen 12d containing the wire(s) 16. The slit 22 originates at the proximal end 12a of the catheter 12, and it can extend along all or only a portion of the remainder of the catheter 12. In an exemplary embodiment, the slit 22 extends along less than about one half of the length *l_{c}* of the catheter 12. This is particularly desirable as it reduces the likelihood of bodily fluids and/or humidity entering through the slit 22 and coming into contact with the sensor 14. It is also desirable to prevent bodily fluids and/or humidity from coming into contact with the wire(s) 16, thus the slit 22 is preferably substantially fluid impermeable in a closed position. That can be achieved by providing a catheter 12 that is formed from a material, such as a silicone rubber, that is self-sealing. Alternatively, or in addition, the slit 22 can include a coating disposed therein to facilitate sealing of the slit 22 when the wire(s) 16 are not extending therethrough. The wire(s) 16 and the sensor 14, as a sub-assembly, can also optionally be coated prior to implantation into the catheter 12 to further protect them from coming into contact with fluids. One example of a suitable material for coating the sub-assembly is Parylene®. A person skilled in the art will appreciate that a variety of other techniques can be used to allow the wire(s) 16 to be removably coupled to the catheter 12.

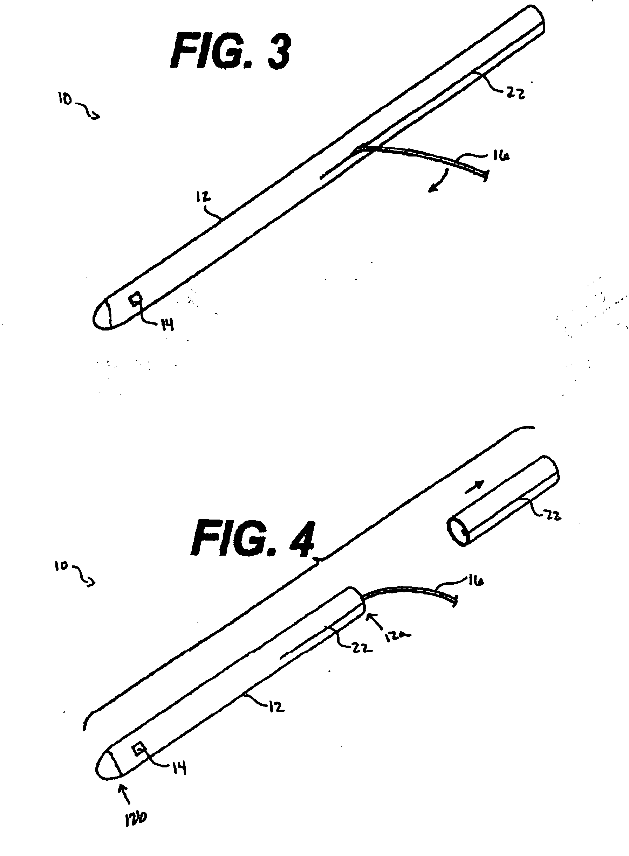

FIGS. 3 and 4 illustrate the device 10 in use. As shown in FIG. 3, once the distal portion 12b of the catheter 12 is implanted in a patient's ventricle (not shown), the wire(s) 16 can be pulled through the slit 22 starting at the proximal end 12a of the catheter 12. The remaining proximal portion 12a of the catheter 12 that does not contain the wire(s) 16 can now be trimmed, e.g., using a cutting device, to a desired length. The wire(s) 16 can then be inserted back into the catheter 12 through the slit 22, as shown in FIG. 4. The proximal end 12a of the catheter 12 is then able to be connected to another device, such as a valve for controlling fluid flow from the ventricle to the fluid drainage site.

The device 10 can be formed from a variety of materials. In an exemplary embodiment, however, the catheter 12 is formed from a flexible, biocompatible material. Suitable materials include, for example, polymers such as silicones, silicone-like materials, such as polyethylene, and polyurethanes. The catheter 12 can also optionally be formed from a radio-opaque material.

One skilled in the art will appreciate further features and advantages of the invention based on the above-described embodiments. Accordingly, the invention is not to be limited by what has been particularly shown and described, except as indicated by the appended claims.

## Claims

1. An implantable fluid management device (10), comprising:
an elongate catheter (12) having a proximal end (12a), a distal end (12b) and a first inner lumen (12c) extending therethrough for connection to an implantable valve device;
a sensor (14) disposed at a distal portion of the catheter (12);
at least one wire (16) having a distal end coupled to the sensor (14) and having a proximal end (16a) that is adapted to mate to an external component (18) for powering and/or communicating with the sensor (14), the at least one wire (16) extending along a length of the catheter (12) such that the at least one wire (16) is in fluid isolation from the inner lumen of the catheter (12), and
**characterised in that** the at least one wire (16) is separable from a proximal portion of the catheter (12) whereby the length of the catheter (12) is selectively adjustable prior to connection to the implantable valve device and independent of the at least one wire (16).

2. The device (10) of claim 1, wherein the at least one wire (16) is disposed within a second lumen (12d) that is isolated from the first lumen (12c).

3. The device (10) of claim 2, further comprising a slit (22) extending through an outer wall of the catheter (12) into the second lumen (12d), the slit (22) extending along at least a portion of a length of the catheter (12) from the proximal end (12a) thereof such that a portion of the at least one wire (16) can be at least partially removed from the catheter (12) through the slit (22) to allow the length of the catheter (12) to be selectively adjusted.

4. The device (10) of claim 1, further comprising a slit (22) extending through an outer wall of the catheter (12) along at least a portion of a length of the catheter (12) from the proximal end (12a) thereof such that a portion of the at least one wire (16) can be at least partially removed from the catheter (12) through the slit (22) to allow the length of the catheter (12) to be selectively adjusted.

5. The device (10) of claim 4, wherein the at least one wire (16) is disposed within a second lumen (12d) that is isolated from the first lumen (12c) and the slit (22) extends into the second lumen (12d).

6. The device (10) of claim 1, wherein the at least one wire (16) is disposed within a secondary catheter (12) that is coupled to the catheter (12) and that can be peeled apart from the catheter (12) to allow the length of the catheter (12) to be selectively adjustable, independent of the length of the second catheter (12).

7. The implantable fluid management device (10) of claim 1 further comprising:
a second inner lumen (12d) extending through the catheter (12) and isolated from the first inner lumen (12c);
the at least one wire (16) extending through the second lumen (12d) in the catheter (12); and
a slit (22) extending through an outer wall of the catheter (12) into the second lumen (12d) along at least a portion of a length thereof such that a portion of the at least one wire (16) can be at least partially removed from the catheter (12) through the slit (22) whereby the length of the catheter (12) is selectively adjustable.

8. The device (10) of any one of claims 2, 3, 5 and 7, wherein the first lumen (12c) has a diameter that is greater than a diameter of the second lumen (12d).

9. The device (10) of any one of claims 2, 3, 5 and 7, wherein the second lumen (12d) is formed with an invagination of the outer wall of the catheter (12) extending within the first lumen (12c).

10. The device (10) of any one of claims 3, 4 and 7, wherein the slit (22) extends along a distance less than the length of the catheter (12).

11. The device (10) of any one of claims 3, 4 and 7, wherein the slit (22) extends along less than about one half of the length of the catheter (12).

12. The device (10) of any one of claims 3, 4, 7, 10 and 11, wherein the slit (22) is substantially fluid impermeable in a closed position.

13. The device (10) of any one of claims 3, 4, 7, 10, 11 and 12, wherein the catheter (12) is made from a material that is self-sealing.

14. The device (10) of any one of claims I to 13, wherein the catheter (12) is formed from a flexible, biocompatible polymer.

15. The device (10) of any one of claims 1 to 14, wherein the catheter (12) is formed from a silicone, silicone-like material or polyurthane polymer.

16. The device (10) of any one of claims 1 to 15, wherein the sensor (14) is disposed with a wall of the catheter (12) such that the sensor (14) is adapted to sense conditions present around the catheter (12).

17. The device (10) of any one of claims I to 16, wherein the sensor (14) is a pressure sensor (14)

18. The device (10) of any one of claims 1 to 17, wherein the sensor (14) has a diameter that is equal to or less than about 3mm.

## Patentansprüche

1. Implantierbare Vorrichtung (10) für das Fluidmanagement, die aufweist:
einen länglichen Katheter (12), der ein proximales Ende (12a), ein distales Ende (12b) und ein erstes inneres Lumen (12c) die sich durch ihn für den Anschluß an eine implantierbare Ventilvorrichtung erstreckt, hat;
einen Sensor (14), der an einem distalen Teil des Katheters (12) angeordnet ist;
wenigstens einen Draht (16) mit einem distalen Ende, das an den Sensor (14) gekoppelt ist, und mit einem proximalen Ende (16a), das so ausgelegt ist, daß es an eine externe Komponente (18) zum Betreiben des Sensors und/oder Kommunizieren mit dem Sensor (14) angepaßt ist, wobei der wenigstens eine Draht (16) sich entlang einer Länge des Katheters (12) derart erstreckt, daß der wenigstens eine Draht (16) von dem inneren Lumen des Katheters (12) fluidisch isoliert ist, und
**dadurch gekennzeichnet, daß** der wenigstens eine Draht (16) von einem proximalen Teil des Katheters (12) trennbar ist, wodurch die Länge des Katheters (12) vor dem Anschluß an die implantierbare Ventilvorrichtung und unabhängig von dem wenigstens einen Draht (16) wahlweise einstellbar ist.

2. Vorrichtung (10) nach Anspruch 1, bei der der wenigstens eine Draht (16) innerhalb eines zweiten Lumens (12d) angeordnet ist, das von dem ersten Lumen (12c) isoliert ist.

3. Vorrichtung (10) nach Anspruch 2, weiter mit einem Schlitz (22), der sich durch eine Außenwand des Katheters (12) in das zweite Lumen (12d) erstreckt, wobei sich der Schlitz (22) entlang wenigstens eines Teiles einer Länge des Katheters (12) von dessen proximalem Ende (12a) derart erstreckt, daß ein Teil des wenigstens einen Drahtes (16) wenigstens teilweise durch den Schlitz (22) aus dem Katheter (12) entfernt werden kann, um zu ermöglichen, daß die Länge des Katheters (12) wahlweise angepaßt wird.

4. Vorrichtung (10) nach Anspruch 1, weiter mit einem Schlitz (22), der sich durch eine Außenwand des Katheters (12) entlang wenigstens einem Teil einer Länge des Katheters (12) von dessen proximalem Ende (12a) derart erstreckt, daß wenigstens ein Teil des wenigstens einen Drahtes (16) wenigstens teilweise durch den Schlitz (22) aus dem Katheter (12) entfernt werden kann, um zu ermöglichen, daß die Länge des Katheters (12) wahlweise angepaßt wird.

5. Vorrichtung (10) nach Anspruch 4, bei der der wenigstens eine Draht (16) innerhalb eines zweiten Lumens (12d) angeordnet ist, das von dem ersten Lumen (12c) isoliert ist, und der Schlitz (22) sich in das zweite Lumen (12d) erstreckt.

6. Vorrichtung (10) nach Anspruch 1, bei der der wenigstens eine Draht (16) innerhalb eines sekundären Katheters (12) angeordnet ist, der an den Katheter (12) gekoppelt ist und von dem Katheter (12) abgemantelt werden kann, um zu erlauben, daß die Länge des Katheters (12) unabhängig von der Länge des sekundären Katheters (12) wahlweise anpaßbar ist.

7. Impantierbare Vorrichtung (10) für das Fluidmanagement nach Anspruch 1, die weiter aufweist:
ein zweites inneres Lumen (12d), das sich durch den Katheter (12) erstreckt und von dem ersten inneren Lumen (12c) isoliert ist;
wobei sich der wenigstens eine Draht (16) durch das zweite Lumen (12d) in den Katheter (12) erstreckt; und
einen Schlitz (22), der sich durch eine Außenwand des Katheters (12) in das zweite Lumen (12d) entlang wenigstens einem Teil dessen Länge derart erstreckt, daß ein Teil des wenigstens einen Drahtes (16) wenigstens teilweise durch den Schlitz (22) aus dem Katheter (12) entfernt werden kann, wodurch die Länge des Katheters (12) wahlweise anpaßbar ist.

8. Vorrichtung (10) nach einem der Ansprüche 2, 3, 5 und 7, bei der das erste Lumen (12c) einen Durchmesser hat, der größer ist als ein Durchmesser des zweiten Lumens (12d).

9. Vorrichtung (10) nach einem der Ansprüche 2, 3, 5 und 7, bei der das zweite Lumen (12d) aus einer Einstülpung der Außenwand des Katheters (12) gebildet ist, die sich in das erste Lumen (12c) erstreckt.

10. Vorrichtung (10) nach einem der Ansprüche 3, 4 und 7, bei der sich der Schlitz (22) entlang einer Entfernung erstreckt, die geringer als die Länge des Katheters (12).

11. Vorrichtung nach einem der Ansprüche 3, 4 und 7, bei der der Schlitz (22) sich entlang weniger als ungefähr einer Hälfte der Länge des Katheters (12) erstreckt.

12. Vorrichtung (10) nach einem der Ansprüche 3, 4, 7, 10 und 11, bei der der Schlitz (22) in einer geschlossenen Position für Fluid im wesentlichen undurchlässig ist.

13. Vorrichtung (10) nach einem der Ansprüche 3, 4, 7, 10, 11 und 12, bei der der Katheter (12) aus einem Material hergestellt ist, das selbstdichtend ist.

14. Vorrichtung (10) nach einem der Ansprüche 1 bis 13, bei der der Katheter (12) aus einem flexiblen, biokompatiblen Polymer gebildet ist.

15. Vorrichtung (10) nach einem der Ansprüche 1 bis 14, bei der der Katheter (12) aus einem Silikon, einem silikonähnlichen Material oder einem Polyurethanpolymer gebildet ist.

16. Vorrichtung (10) nach einem der Ansprüche 1 bis 15, bei der der Sensor (14) an einer Wand des Katheters (12) derart angeordnet ist, daß der Sensor (14) dazu ausgelegt ist, Bedingungen abzufühlen, die um den Katheter (12) herum vorliegen.

17. Vorrichtung (10) nach einem der Ansprüche 1 bis 16, bei der der Sensor (14) ein Drucksensor (14) ist.

18. Vorrichtung (10) nach einem der Ansprüche 1 bis 17, bei der Sensor (14) einen Durchmesser hat, der gleich oder geringer als ungefähr 3 mm ist.

## Revendications

1. Dispositif implantable de gestion de fluide (10), comprenant :
■ un cathéter allongé (12) ayant une extrémité proximale (12a), une extrémité distale (12b) et un premier lumen interne (12c) s'étendant au travers de celui-ci pour le raccordement à un dispositif de soupape implantable ;
■ un capteur (14) disposé dans une partie distale du cathéter (12) ;
■ au moins un fil (16) ayant une extrémité distale couplée au capteur (14) et ayant une extrémité proximale (16a) qui est adaptée pour être accouplée à un composant externe (18) pour le mettre sous tension et/ou le faire communiquer avec le capteur (14), le au moins un fil (16) s'étendant le long d'une longueur du cathéter (12) de telle sorte que le au moins un fil (16) est isolé par du fluide du lumen interne du cathéter (12), et
**caractérisé en ce que** le au moins un fil (16) peut être séparé d'une partie proximale du cathéter (12), moyennant quoi la longueur du cathéter (12) est ajustable sélectivement avant le raccordement au dispositif de soupape implantable et indépendamment du au moins un fil (16).

2. Dispositif (10) selon la revendication 1, **caractérisé en ce que** le au moins un fil (16) est disposé dans un second lumen (12d) qui est isolé du premier lumen (12c).

3. Dispositif (10) selon la revendication 2, comprenant en outre une fente (22) s'étendant à travers une paroi externe du cathéter (12) vers le second lumen (12d), la fente (22) s'étendant le long d'une partie de la longueur du cathéter (12) depuis l'extrémité proximale (12a) de celui-ci de telle sorte qu'une partie du au moins un fil (16) peut être au moins partiellement enlevée du cathéter (12) à travers la fente (22) pour permettre l'ajustement sélectif de la longueur du cathéter (12).

4. Dispositif (10) selon la revendication 1, comprenant en outre une fente (22) s'étendant à travers une paroi externe du cathéter (12) le long d'au moins une partie d'une longueur du cathéter (12) depuis l'extrémité proximale (12a) de celui-ci de telle sorte qu'une partie du au moins un fil (16) peut être au moins partiellement enlevée du cathéter (12) à travers la fente (22) pour permettre l'ajustement sélectif de la longueur du cathéter (12).

5. Dispositif (10) selon la revendication 4, **caractérisé en ce que** le au moins un fil (16) est disposé dans un second lumen (12d) qui est isolé du premier lumen (12c) et la fente (22) s'étend dans le second lumen (12d).

6. Dispositif (10) selon la revendication 1, **caractérisé en ce que** le au moins un fil (16) est disposé dans un cathéter auxiliaire (12) qui est couplé au cathéter (12) et qui peut être détaché du cathéter (12) pour permettre l'ajustement sélectif de la longueur du cathéter (12), indépendamment de la longueur du second cathéter (12).

7. Dispositif de gestion de fluide implantable (10) selon la revendication 1 comprenant en outre :
■ un second lumen interne (12d) s'étendant à travers le cathéter (12) et isolé du premier lumen interne (12c) ;
■ le au moins un fil (16) s'étendant à travers le second lumen (12d) dans le cathéter (12) ; et
■ une fente (22) s'étendant à travers une paroi externe du cathéter (12) vers le second lumen (12d) le long d'au moins une partie de la longueur de celui-ci de telle sorte qu'une partie du au moins un fil (16) peut être au moins partiellement enlevée du cathéter (12) à travers la fente (22), moyennant quoi la longueur du cathéter (12) est sélectivement ajustable.

8. Dispositif (10) selon l'une quelconque des revendications 2, 3, 5 et 7, **caractérisé en ce que** le premier lumen (12c) a un diamètre qui est supérieur au diamètre du second lumen (12d).

9. Dispositif (10) selon l'une quelconque des revendications 2, 3, 5 et 7, **caractérisé en ce que** le second lumen (12d) est formé par invagination de la paroi externe du cathéter (12) s'étendant dans le premier lumen (12c).

10. Dispositif (10) selon l'une quelconque des revendications 3, 4 et 7, **caractérisé en ce que** la fente (22) s'étend le long d'une distance inférieure à la longueur du cathéter (12).

11. Dispositif (10) selon l'une quelconque des revendications 3, 4 et 7, **caractérisé en ce que** la fente (22) s'étend le long d'environ moins de la moitié de la longueur du cathéter (12).

12. Dispositif (10) de l'une quelconque des revendications 3, 4, 7, 10 et 11, **caractérisé en ce que** la fente (22) est sensiblement imperméable au fluide en position fermée.

13. Dispositif (10) selon l'une quelconque des revendications 3, 4, 7, 10, 11 et 12, **caractérisé en ce que** le cathéter (12) est fait d'un matériau qui est auto-obturant.

14. Dispositif (10) selon l'une quelconque des revendications 1 à 13, **caractérisé en ce que** le cathéter (12) est formé d'un polymère flexible, biocompatible.

15. Dispositif (10) selon l'une quelconque des revendications 1 à 14, **caractérisé en ce que** le cathéter (12) est formé d'un matériau de silicone, semblable à du silicone ou de polymère polyuréthane.

16. Dispositif (10) selon l'une quelconque des revendications 1 à 15, **caractérisé en ce que** le capteur (14) est disposé sur une paroi du cathéter (12) de telle sorte que le capteur (14) est adapté pour détecter les conditions présentes autour du cathéter (12).

17. Dispositif (10) selon l'une quelconque des revendications 1 à 16, **caractérisé en ce que** le capteur (14) est un capteur de pression (14).

18. Dispositif (10) selon l'une quelconque des revendications 1 à 17, **caractérisé en ce que** le capteur (14) a un diamètre égal ou inférieur à environ 3 mm.
